(19) Europäisches Patentamt
European Patent Office
Office européen des brevets

(11) Veröffentlichungsnummer: **0 014 238**
**A2**

(12) # EUROPÄISCHE PATENTANMELDUNG

(21) Anmeldenummer: 79104663.4

(22) Anmeldetag: 23.11.79

(51) Int. Cl.³: A 61 K 9/02
A 61 K 9/06

(43) Veröffentlichungstag der Anmeldung:
20.08.80 Patentblatt 80/17

(84) Benannte Vertragsstaaten:
BE CH DE FR GB IT LU NL SE

(71) Anmelder: Posthuma, Albert E.
3520 Burton Ridge
Grand Rapids, Mich. 49506(US)

(71) Anmelder: Woodhouse, Robert C.
1664 Alexander
Grand Rapids Mich. 49506(US)

(72) Erfinder: Posthuma, Albert E.
3520 Burton Ridge
Grand Rapids, Mich. 49506(US)

(72) Erfinder: Woodhouse, Robert C.
1664 Alexander
Grand Rapids Mich. 49506(US)

(74) Vertreter: Krieger, Hans Jochen, Dr. et al,
Rechtsanwälte KRIEGER . ROTHE . PINDER
Gogrevestrasse 11-13
D-4000 Düsseldorf(DE)

(54) **Schleimbildendes synthetisches Schmier- und Gleitmittel, insbesondere für den Vaginalbereich, und dessen Verwendung im Bereich der diagnostischen, chirurgischen und behandelnden Medizin.**

(57) Die Erfindung umfasst ein neues synthetisches, physiologisches schleimbildendes Schmier- und Gleitmittel insbesondere zur Verwendung im Vaginalbereich, in der Medizin und in der Chirurgie.

Das Mittel basiert auf einer verdünnten Lösung von hochpolymerem anionischem Polyacrylamid in Wasser. Die Lösung ist klar, von mittlerer Viskosität, nicht toxisch, steril und kann zusätzlich Konservierungsmittel, Bakteriostatika, Arzneimittel und Stabilisatoren enthalten.

Die Lösung dient als Ersatz für menschlichen Schleim im Vaginalbereich, als Schmier- und Gleitmittel für medizinische Instrumente, insbesondere solchen, die in den Körper eingeführt werden (Katheter, Tracheoskope, Endoskope) als Gleitmittel für Suppositorien, als filmbildendes und wieder reaktivierbares Konservierungsmittel anderer chirurgischer Instrumente sowie als Medium und Träger für Arzneimittel allgemein.

Die neuen Schmier- und Gleitmittel sind lagerbeständig und können bezüglich pH-Wert und Viskosität in weiten Bereichen variiert werden.

EP 0 014 238 A2

Croydon Printing Company Ltd

BEZEICHNUNG GEÄNDERT
siehe Titelseite

Schleimbildendes synthetisches Schmier- und Gleitmittel
insbesondere für den Vaginalbereich.

Beschreibung und Beispiele

Die Erfindung betrifft ein synthetisches, physiologisches, schleimbildendes Schmier- und Gleitmittel mit ganz spezifischen Eigenschaften zur Verwendung im Vaginalbereich, in der Medizin und hier besonders auch in der Chirurgie.

Basis dieses neuen Mittels ist eine schleimartige Stoffe absondernde bzw. liefernde wässrige Lösung eines hochmolekularen Polyacrylamids.

Bestimmte medizinische und physiologische Erscheinungen bzw. Veränderungen am oder im menschlichen Körper erfordern bei der Behandlung, Beobachtung, Eingriffen usw. den Einsatz von schleimartigen Schmier- oder Gleitmitteln. Dies gilt insbesondere für den Vaginalbereich der Frau, aber auch für den Einsatz chirurgischer Instrumente in Körperöffnungen, usw., wo z.B. bei Untersuchungen des After- und Mastdarmbereichs, bei Luftröhren-, Speiseröhren-, Magen- und Darmspiegelungen die Anwendung geeigneter Schmier- und Gleitmittel unerlässlich sind.

Von besonderer Bedeutung sind diese Gleitmittel -wie bereits oben gesagt- für den Vaginalbereich der Frau

z.B. bei Schwunderscheinungen als Folge der Menopause oder der Hysterektomie oder auch ganz allgemein bei ungenügender oder völlig eingestellter Erzeugung notwendiger Mengen an Schleimstoffen durch den menschlichen Organismus.

Es wurden bereits eine größere Anzahl insbesondere synthetischer Ersatzstoffe für die vom Organismus erzeugten Schleime vorgeschlagen. So sind z.B. verschiedene Gele organisch synthetischer oder pflanzlicher Natur, entsprechende Gelees, Emulsionen, aber auch Stoffe wie Glykole (z.B. Propylenglykol) Glycerin und Glyceride, sehr reine Kohlenwasserstoffe, Cellulosederivate wie Methylcellulose, Carboxymethyl- und Äthylcellulose, Petrolatum, gesättigte und ungesättigte langkettige Carbonsäuren (neutral) usw. aus dem Angebot derartiger Stoffe zu nennen.

Allen diesen Stoffen und Stoffkombinationen ist eigen, daß sie nicht speziell für die Humanmedizin, für die Anwendung im Bereich des menschlichen Körpers usw. entwickelt wurden, sondern es erfolgte vielfach eine Auswahl, die sich nur aus den Eigenschaften als Schmiermittel oder Gleitmittel oder auch Emulgier- und Verdickungsmittel herleiten läßt, während die hohen Anforderungen an Verträglichkeit, Toxizität, körpernahes Verhalten u.a. außer acht bleiben.

Die dem vom Oranismus erzeugten Schleim, ob im Vaginalbereich oder in den Luft-, Speisen-, Harn-, und Verdauungswegen, eigenen Qualitäten werden von bekannten Schmier- und Gleitstoffen bei weitem nicht erfüllt.

Aufgabe der Erfindung ist es daher, ein neues schleimbildendes, synthetisches, leicht zugängliches Schmier- und Gleitmittel zu schaffen, das alle Nachteile bekannter Schmier- und Gleitmittel ausschaltet, das ein dem natürlichen, vom Organismus produzierten Schleim ähnliches, wenn nicht sogar gleiches Verhalten, in chemischer, physikalischer und physiologischer Hinsicht aufweist und das somit in praktisch allen Einsatzbereichen der vom Organismus erzeugten Schleime unbedenklich verwendet werden kann.

Außerdem vermittelt das neue Schmier- und Gleitmittel gegenüber bekannten Mittel dieser Art eine wesenlich höhere Schlüpfrigkeit, gesteigerte Adhäsionswirkung und Klebrigkeit und schließlich eine außerordentlich hohe Stabilität beim Lagern sowie langanhaltende Wirkung nach der Anwendung bzw. der topischen Applikation.

Damit wird erstmals ein Schmier- und Gleitmittel für den Einsatz im Humanbereich, insbesondere im Vaginalbereich, für die Verbesserung der Gleitwirkung chirurgischer Instrumente, für Suppositorien, für Gummihandschuhe und Fingerlinge bei Tastbehandlungen im After usw. geschaffen, das nicht mehr als Einsatz- oder Hilfs- und Zusatzmittel für natürlichen Schleim zu werten ist, sondern ein diesem praktisch gleichwertiges Produkt darstellt.

Dies hat sich im Verlaufe einer Vielzahl von Untersuchungen sowohl bei Eingriffen im menschliche Körper (Katheter, Spiegelungen, Kanülen uaw.) als auch beim direkten Schleimersatz im Vaginalbereich erwiesen.

Das neue Mittel verbindet die Eigenschaften der mit natürlichem, vom Organismus erzeugten Schleimen verbundenen Schmier- und Gleitwirkung mit einer ausgezeichneten Haft- und Klebeaktivität, Schlüpfrigkeit, bakteriostatischer und stofflicher Stabilität und Immunität, hoher Filmbildungstendenz und Schleimwirkung.

Die insbesondere bei Cellulosederivaten zu beobachtende Ausbildung von faserigen Partikeln scheidet völlig aus.

Der $P_H$-Wert des neuen Schmier- und Gleitmittels kann innerhalb eines Bereichs von 3,0 - 10,0 ohne Veränderung der Qualität und der physiologischen Eigenschaften beliebig variiert werden, wodurch ein sehr breites Anwendungsspektrum gegeben ist.

Auch besitzt das neue Schmier- und Gleitmittel keinen Geruch, es erzeugt keinen Geschmack, es besitzt praktisch keinerlei toxische Nebenwirkungen, es kann auf einfachste Weise steril gemacht werden und zeigt schon bei Zugabe von kleinsten Mengen bakterizider Stoffe praktisch vollkommene Haltbarkeit.

Das neue Schmier- und Gleitmittel ist auch hervorragend geeignet als Medium oder Träger für zusätzlich zur Schleimwirkung einzubringende Arzneimittel, sodaß eine Doppelfunktion erfüllt wird. Übliche, insbesondere wasserlösliche Arzneistoffe, aber auch in wässrigem Medium dispergier- und emulgierbare Wirkstoffe, können ohne Bedenken und ohne Minderung der Gleitwirkung des Mittels selbst zugesetzt werden.

Weiter ist das neue Schmier- und Gleitmittel als Trocken- und damit als Korrosionsschutzmittel für chirurgische Instrumente hervorragend geeignet. Durch Aufsprühen oder im Tauchverfahren auf die Oberfläsche von Scheren, Zangen, Pinzetten, Pipetten, Skalpellen usw. aufgebrachtes Schmier- und Gleitmittel gemäß der Erfindung gibt schon bei Zimmertemperatur sein Wasser schnell ab -wenig erhöhte Zimmertemperatur oder die Anwendung von Ventilatoren und Gebläsen ist zweckmäßig- und hinterläßt einen voll abdeckenden stabilen Film aus Polyacrylamid.

Beim Gebrauch des behandelden Instruments genügt ein leichtes Anfeuchten mit einer Sprühflasche oder einer Düse oder auch schon erneutes Eintauchen in Wasser, um eine Oberfläsche zu schaffen, die entweder als solche eine hohe Gleit- und Schmierwirkung zeigt oder durch einfaches Abwischen mit einem Lappen usw. gereinigt werden kann.

Auch kann der dieser Art aufgetragene und wiedererzeugte Gleitmittelbelag z.B. bei Kathetern direkt als Gleitmittel wirken.

Das neue Schmier- und Gleitmittel mit den physiologischen, chemischen und physikalischen Eigenschaften natürlichen Schleims besteht aus einer wässrigen Lösung von Polyacrylamid. Dieses Polyacrylamid wird bekanntlich durch Polymerisation des monomeren Acrylamids $[CH_2=Ch-\underset{\underset{O}{\|}}{C}-NH_2]$ gewonnen.

Das der Erfindung zugrunde liegende Polyacrylamid hat ein Molekulargewicht zwischen etwa 3 000 000 und etwa 5 000 000 entsprechend einem Polymerisationsgrad von ca. 43 000 bis etwa 72 000.

Das Produkt ist innerhalb geeigneter Grenzen wasserlöslich.

An die Auswahl des Polymerisationsgrades muß die Forderung gestellt werden, daß nur ein solches Polymerisat verwendet wird, welches eine semipermeable Membran mit den Eigenschaften des Darms oder der Haut nicht passieren kann. Diese Forderung ist notwendig, damit das Material die Haut nicht durchdringt und keine sperminaktivierende Eigenschaft ausbildet.

Um die Anforderungen üblicher Spezifikationen der Gesundheitsämter usw. zu erfüllen, muß aus Gründen der Toxizität darauf geachtet werden, daß der Gehalt an Monomeren im Polyacrylamid unter etwa 0,05 Gew.-% liegt.

Diese allgemeinen Auflagen erfüllend, wird bei der Herstellung eine klare, farblose, wässrige Lösung erhalten, die nach bekannten Verfahren, z.B. im Autoklaven, sterilisiert werden kann.

Die handelsüblichen hochmolekularen Polyacrylamide sind im allgemeinen leicht bis mittel anionischer Natur, was auf eine gewisse, anwendungstechnisch bedingte Modifizierung zurückzuführen ist. Nichtmodifiziert reagiert Polyacrylamid dagegen völlig nichtionisch bzw. indifferent.

Normalerweise sind aber auch beim nicht modifizierten Produkt Anteile von weniger als 0,5 % der Amidgruppen zu anionisch reagierenden Carboxylgruppen hydrolysiert. Die angestrebte Modifizierung wird durch einen weiteren Ersatz von Amidgruppen durch Carboxylgruppen und deren Neutralisation durch Alkalimetall- insbes. Natrium-Ionen erreicht. Hierdurch wird zwangsläufig die anionische Natur des hochpolymeren Acrylamids verstärkt.

Das Polyacrylamid ist dann durch folgende Molekülfolge ausgezeichnet:

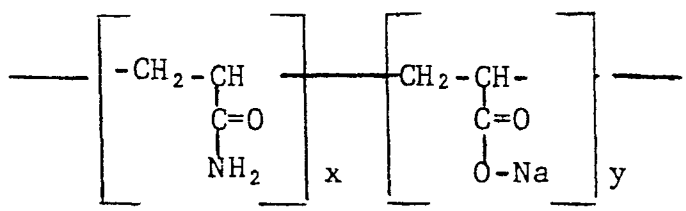

Bei einem bekannten handelsüblichen Produkt, das sich im Rahmen der Erfindung besonders bewährt hat, sich auf diese Weise zwischen 26,0 und 36,0 % der Amidgruppen (x) durch Carboxylatgruppen (y) ersetzt, sodaß eine Vielzahl hydrophiler, insbesondere Natriumionen enthaltende Gruppen ausgebildet werden, während der Rest (x) aus reinen Amidgruppen besteht.

Hieraus leitet sich die hohe und für das neue Schmier- und Gleitmittel wichtige Wasserlöslichkeit ab.

Polyacrylamide dieser Art sind im trockenen, zerkleinerten Zustand als weiße, freifließende, nicht klebende anionische Pulver marktgängig.

Es hat sich als zweckmäßig erwiesen, entweder dem Polyacrylamid-Pulver vor der Bereitung der wässrigen Lösung

oder zusammen mit diesem bei der Bereitung der wässrigen Lösung ein geeignetes Konservierungsmittel einzusetzen, um die Lagerbeständigkeit der Lösung und ein antibakterizides verhalten bei der Anwendung zu erhöhen bzw. zu erzielen.
Besonders geeignete Konservierungsmittel sind -obwohl auch andere bekannte Mittel dieser Art durchaus verwendet werden können- z.B. Methyl-p-Hydroxybenzoat, Propyl-p-Hydroxybenzoat, Salicylanilide wie 4,5-Dibrom-Salicylanilid, und Benzalkoniumchlorid.

Wichtig ist, daß das zugesetzte Konservierungsmittel selbst keine epidermalen und die Schleimbildung ungünstig beeinflußenden Eigenschaften aufweist. Im übrigen gelten auch für das Konservierungsmittel die Anforderungen bezüglich Toxizität, $p_H$-Stabilität usw.

Die Konzentration des Polyacrylamids in Wasser liegt im allgemeinen zwischen 0,10 und 5,00 Gew.-%. Sie richtet sich nach der gewünschten Schmier- und Gleitwirkung, nach der Viskosität, der Haftwirkung und der Schlüpfrigkeit des Endprodukts.

Wichtig ist für den jeweiligen Anwendungsbereich eine optimale Viskosität, damit die Gleitwirkung voll zur Geltung kommt. Andererseits sind zu hohe Konzentrationen und damit Viskositäten insbesondere bei der Anwendung im Vaginalbereich unerwünscht. In diesem speziellen Falle sind Viskositäten zwischen etwa 1000 und 3000 cp besonders geeignet. In bestimmten Fällen können Viskositäten bis etwa 20 cp noch zweckmäßig sein, dagegen sind Viskositäten über 3000 cp wenig brauchbar.

Liegen Lösungen mit niedrigen Konzentrationen vor, so

ist durch nachträgliche Zugabe von weiterem Polyacrylamid eine Zunahme der Konzentration durchaus möglich. Umgekehrt können konzentrierte Lösungen durch Verdünnen mit Wasser auf einfachste Weise in verdünntere Lösungen übergeführt werden.

Hierin liegt ein weiterer besonderer Vorteil der Verwendung von Polyacrylamid in wässriger Lösung als Schmier- und Gleitmittel im Sinne der Erfindung. Er können so z.B. aus einem Stammkonzentrat beliebige verdünntere Lösungen gewonnen werden, deren Konzentration auf ganz spezielle Anwendungsbereiche abgestimmt ist.

Für erhöhte Viskositäten können auch bekannte, nichttoxische und physiologisch verträgliche viskositätserhöhende Zusätze anderer Natur eingearbeitet werden.

Der Gehalt an Konservierungsmitteln liegt im allgemeinen zwischen etwa 15 und 30 %, bezogen auf die vorhandene Menge Polyacrylamid.

Der $p_H$-Wert der als Schmier- und Gleitmittel geeigneten lösung von Polyacrylamid in Wasser beträgt etwa 3,0 bis 10,0. Er richtet sich selbstverständlich nach dem Anwendungszweck und kann dieserhalb innerhalb dieses Bereichs variiert werder. Bei eienr Anwendung im Vaginalbereich sollte der $p_H$-Wert bei etwa 5,0 liegen. Für allgemeine Anwendungen ist die Einstellung auf einen $p_H$ von etwa 5,0 bis etwa 8,0 empfehlenswert.

Die Einstellung des gewünschten oder erforderlichen $p_H$-Werts erfolgt durch Zusatz eines geeigneten sauer reagierenden Stoffes, insbesondere durch Zitronensäure oder einer anderen schwachen organischen Säure.

Es empfiehlt sich, kurz vor Gebrauch, den $p_H$-Wert potentiometrisch oder mit einem Indikator festzustellen und dann durch Säurezusatz zu regulieren.

Die im allgemeinen zweckmäßigste Konzentration der Lösung an Polyacrylamid liegt bei 0,30 bis 1,50 Gew.-% bzw.g je 100 ml Wasser.

Bei höheren Konzentrationen und damit weniger fließfähigen Lösungen können diese gelegentlich eine gelartige Form annehmen, die nicht nur bei der Herstellung dadurch Schwierigkeiten bereitet, daß eine vollständige Dispersionsbildung nur noch bedingt erreicht wird, sondern es wird auch der Anwendungsbereich eingeschränkt.

Am brauchbarsten erwiesen sich Lösungen für den Vaginalbereich und für die Anwendung als Gleitmitel bei chirurgischen Instrumenten, die etwa 0,75 g Polyacrylamid auf 100 ml $H_2O$ enthalten.

Eine entsprechende Standardzusammensetzung des neuen Schmier- und Gleitmittels hat z.B. folgenden Aufbau:

37,5 g anionisch reagierendes Polyacrylamid;
8,5 g Konservierungsmittel (z.B. Methyl-p-Hydroxybenzoat)
5000,0 ml Wasser.

Durch Zugabe einer kleinen Menge Zitronensäure wird der $p_H$-Wert dieses Ansatzes auf z.B. 5,0 eingestellt.

Die Herstellung der Lösung erfolgt zweckmäßig unter Zuhilfenahme eines geeigneten Rührwerks, das schnelllaufend in kurzer Zeit die völlige Lösung herbeiführt. Hierfür sollten im allgemeinen nicht mehr als 2 - 3 Minuten erforderlich sein, da bei längerer Rührdauer die Polyacrylamid-Partikel zum Zusammenballen neigen. Nur durch zeitraubendes Dispergieren mit hoher Rürgeschwindigkeit lassen sich diese Agglomerate wieder brechen und in eine klare Lösung überführen.

Eine gute Hilfe zur schnellen Lösungsbildung ohne Agglomeration ist die Verwendung von siedendem Wasser in Gegenwart eines Turborührers oder eines anderen schnell bewegten Systems. Dabei werden die Polyacrylamid-Partikel (Pulver, Granulat uw..) unter Rühren in siedendes Wasser eingetragen und die Temperatur allmählich auf Zimmertemperatur herabgesetzt.

Wie bereits führer gesagt, eignet sich die Lösung von Polyacrylamid in Wasser auch hervorragend als Träger bzw. Medium für andere Arzneimittel, die in geringen Mengen der Lösung zugesetzt werden. Besonders geeignet sind hier Antibiotika, Anästetika, Analgetika, Desinfektionsmittel, Antidepressiva usw., die beim Einsatz des neuen Mittels als Schmier- und Gleitstoff gleichzeitig ihre Wirkung entfalten und dabei z.B. schmerzstillend, narkotisierend, desinfizierend, entkrampfend usw. wirken.

Obwohl die nachfolgenden Betrachtungen nicht in engerem Zusammenhang mit dem Gegenstand der Erfindung zu sehen sind, sei noch darauf verwiesen, daß auch andere polymere Stoffe neben Polyacrylamid als Schmier- und Gleitmittel untersucht wurden. Insbesondere richtete sich

diese Untersuchung auf den Einsatz von wässrigen Lösungen von Polyäthylen als Alternative für Lösungen von Polyacrylamid.

Dabei stellte sich heraus, daß zunächst nur mit Polyacrylamid glasklare, wässrige Lösungen vorstehender geeigenter Konzentration und Viskosität herstellbar sind, während entsprechende Lösungen von Polyäthylen eine starke Tendenz zu Eintrübungen zeigen, die vom Patienten nicht ohne erheblichen Widerspruch akzeptiert werden. Darüberhinaus treten beim Lagern derartiger Polyäthylenlösungen Aussalzungseffekte auf, die zu häßlichen Abscheidungen, Schlierenbildung und nicht mehr resolvierbaren Bodenkörpern, verbunden mit Konzentrationsänderungen, führen.

Auch viele andere Wirkungen des Polyacrylamids werden vom Polyäthylen nicht erfüllt. So sind einmal die auf chriurgischen Instrumenten abgeschiedenen bzw. nach dem Verdampfen des Wassers ausgebildeten Filme nicht mehr in die gewünschte Schmier- und Gleitkonsistenz rückführbar. Weiter könen Polyäthylenlösungen der erforderlichen Konzentration durch Autoklavenbehandlung nicht sterilisiert werden, sodaß die umständliche und kostspielige Gassterilisierung erforderlich wird.

Es versteht sich, daß weitere Anwendungsgebiete des neuen, auf der Basis von Polyacrylamid in wässriger Lösung aufgebauten Schmier- und Gleitmittels gegeben sind, die nicht alle im Rahmen dieser Beschreibung genannt werden können.

Die Lösungen können selbstverständlich auch angefärbt, mit Geruchs- und Geschmacksstoffen versehen oder auf

sonstige bekannte Weise in eine für den Patienten angnehme Form und Aussehen übergeführt werden, solange die pharmakologische bzw. physiologische Wirkung hierdurch nicht nachteilig beeinflußt wird.

Derartige Zusätze sind besonders geeignet beim Einsatz des neuen Schmier- und Gleitmittels für Suppositorien, bei der Schlüpfrigmachung von Kathetern, Tracheoskopen, Endoskopen usw.

Auch können Arzneimittel, Farb- und Duftstoffe solchen Polyacrylamidlösungen zugestzt werden, die zur sterilen Aufbewahrung und nach Wassereinwirkung erneuten Aktivierung von beschichteten Instrumenten (unter Filmbildung) verwendet wurden.

Die Vielzahl von Einsatzgebieten der neuen, schleimbildenden, synthtischen Schmier- und Gleitmittellösung aus Polyacrylamid in Wasser ergibt sich aus den vorstehend nur beispielhaft dargelegten Möglichkeiten, ohne daß durch diese speziellen Angaben die allgemeine Verwertbarkeit des neuen Schmier- und Gleitmittels erschöpft sei.

Einzelheiten über Herstellung und Eigenschaften des neuen Schmier- und Gleitmittels werden in den nachfolgenden Beispielen beschrieben:

Beispiel 1

9,0 g Polyacrylamid (Pulver, Granulat, Chips oder. dergl.), das ca. 26 - 36 % anionische Gruppen aufweist und z.B. unter dem Handelsnamen "Separan" angeboten wird, werden zusammen mit 1,8 g Mehtyl-p-hydroxybenzoat und 0,04 g 4,5-Dibromosalicylanilid in 1200 ml Wasser dispergiert. Dies kann mit Hilfe bekannter Schnellrührer, Emulgatoren usw. bei Zimmertemperatur oder besser in siedendem Wasser mit nachfolgender Erkaltung auf einfachste Weise erfolgen.

Unter weiterem Rühren wurde durch Zugabe einer wässrigen Lösung von Zironensäure der $p_H$-Wert auf 5,0 eingestellt.

Die erhaltene klare und keine Rückstände aufweisende Lösung bzw. Dispersion erfüllt alle Anforderungen eines hochwirksamen, nicht toxischen, gut klebenden und haftenden schleimartigen Schmier- und Gleitmittel.

Durch Tests mit Fingerauftragung wurde auch die hervorragende Wirkung in der Scheide beobachtet und bestätigt.

Beispiel 2

Das nach Beispiel 1 erhaltende Produkt wurde als Gleitmittel bei der Einführung eines Katheters in den menschlichen Körper verwendet. Hierzu wurde das Produkt in dünner Schicht auf das Katheter aufgetragen und letzteres sofort eingeführt. Die Einführung erfolgte ohne besonderen Kraftaufwand, ohne die sonst üblichen Schwierigkeiten und ohne Schmerzempfinden des Patienten.

Bespiel 3

Das nach Beispiel 1 erhaltene Produkt wurde zur Konservierung und Lagerung verschiedener, später in der chirurgie eingesetzter Instrumente verwendet. Insbesondere wurde dabei die hervorragende konservierende, dann nach längerem Lagern durch Berührung mit Wasser oder Körperflüssigkeit wieder reaktivierte Wirkung als Gleit- und Schmiermittel bei Kathetern, Kanülen, Operationsinstrumenten, Handschuhen und Fingerlingen usw. beobachtet.

Hierzu wurden im einzelnen z.B. eine Vielzahl von Kathetern mit dem Produkt nach Beispiel 1 beschichtet und das Wasser der Beschichtung durch Trocknen an der Luft bei etwa 25 - 30°C ausgetrieben. Anschließend wurden die Katheter in bekannter Weise verpackt und längere Zeit gelagert.

Im Einzelfalle wurde nach 4 Wochen die Verpackung entfernt, das Katheter durch Aufsprühen von Wasser aus einer Düse bzw. Spritzflasche angefeuchtet und sofort die Raktivierung des Gleit- und Schmiermittels beobachtet.

Das Katheter wurde unmittelbar nach der Anfeuchtung eingesetzt und zeigte eine hervorragende Gleitung ohne besondere Schmerzempfindungen des Patienten bei der Kathetereinführung. Dabei wurde kein besonderer Zeitaufwand für das Anfeuchten des Katheters gemessen, vielmehr konnte dieses -wie bereits gesagt- sofort eingesetzt werden.

Gleiche Ergebnisse wurden auch an Kanülen, Instrumenten, Gummihandschuhen usw. beobachtet.

Patentansprüche

1.
Schleimbildendes synthetisches Schmier- und Gleitmittel insbesondere für den vaginalen Bereich,

dadurch gekennzeichnet, daß es aus einer bakteriostatischen Lösung von anionischem Polyacrylamid des Molekulargewichts etwa 3 000 000 bis etwa 5 000 000 in einer Konzentration von etwa 0,10 bis etwa 5,00 Gew.-% in Wasser besteht und einen $p_H$-Wert zwischen etwa 3,0 und etwa 10,0 aufweist.

2.
Mittel nach Anspruch 1,
dadurch gekennzeichnet, daß das Polyacrylamid zwischen etwa 26,0 und etwa 36,0 % anionische Gruppen aufweist.

3.
Mittel nach Ansprchen 1 oder 2,
dadurch gekennzeichnet, daß das Polyacrylamid weniger als 0,05 % monomere Gruppen enthält.

4.
Mittel nach Ansprüchen 1 - 3,
dadurch gekennzeichnet, daß es ein Konservierungsmittel enthält.

5.
Mittel nach Anspruch 4,
dadurch gekennzeichnet, daß es als Konservierungsmittel einen Stoff aus der Gruppe Methyl-p-hydroxybenzoat, Propyl-p-hydroxybenzoat, 4,5-Dibrom-salicylanilid und Benzalkoniumchlorid enthält.

6.
Verwendung von Mitteln nach Ansprüchen 1 - 5 als Schleimstoff im Vagnialbereich der Frau.

7.
Verwendung von Mitteln nach Ansprüche 1 - 5 zur Verbesserung der Gleitfähigkeit, Schlüpfrigkeit und der konservierten Lagerfähigkeit medizinischer, insbesondere chirurgischer Instrumente.